# EUROPÄISCHE PATENTANMELDUNG

(11) **EP 1 228 696 A1**
(43) Veröffentlichungstag der Anmeldung: **07.08.2002**
(21) Anmeldenummer: 02001066.6
(22) Anmeldetag: 22.01.2002
(51) Int. Cl.: A23K 1/16, A23K 1/18

(54) **Sorbinsäurepräparat enthaltend Probiotika als Futtermittelzusatz in der Nutztieraufzucht**

(30) Priorität: 05.02.2001 DE 10105347
(71) Anmelder: Nutrinova Nutrition Specialties & Food Ingredients GmbH, 65929 Frankfurt am Main (DE)
(72) Erfinder: Raczek, Nico, Dr., 65779 Kelkheim (DE)
(74) Vertreter: Schweitzer, Klaus, Dr.

(57) **Zusammenfassung**

Die vorliegende Erfindung betrifft ein Präparat/Kit zum Einsatz in Tierfuttermitteln. Das Präparat beziehungsweise das Futtermittelzusatzkit enthält Sorbinsäure und mindestens eine Kultur probiotisch wirkender Mikroorganismen. Weiterhin betrifft die Erfindung die Verwendung des Präparates/Kits allein in Futtermitteln oder in Abmischung mit anderen Futtermittelzusatzstoffen zur Verbesserung des hygienischen Status des Futters und zur Leistungsförderung in der Nutztieraufzucht.

## Beschreibung

Die Erfindung betrifft ein Präparat, welches Sorbinsäure und Probiotika enthält und allein in Futtermitteln oder in Abmischung mit anderen Futtermittelzusatzstoffen in der Nutztieraufzucht angewendet werden kann.

In der Humanernährung werden Probiotika als lebensfähige Mikroorganismen definiert, die bei ausreichender oraler Aufnahme gesundheitsfördernde Effekte haben (J. Nutr. 130: 384S-390S, 2000, M.E. Sanders).

In den letzten Jahren wurde den Probiotika für die Tierernährung wieder mehr Aufmerksamkeit geschenkt. Für Probiotika wird allgemein die Definition von R. Fuller (Journal of Applied Bacteriology 1989, 66, 365-378) herangezogen, wonach es sich um Mikroorganismen handelt, die als Futterzusatzstoff appliziert werden und die aufgrund einer "Unterstützung des Gleichgewichtes" der Darmflora günstige Effekte für das Wirtstier haben. Die Formulierung dieser Definition zeigt deutlich, wie wenige über die Mechanismen bekannt ist, die der Probiotikawirkung zugrunde liegen.

Im Bereich der Tierfutter werden häufig Antibiotika als Leistungsförderer verwendet. Die Anwendung von Antibiotika in diesem Bereich steht im Verdacht für Gefahren, welche von resistenten Bakterien ausgehen, die auch die menschliche Gesundheit langfristig gefährden können, verantwortlich zu sein. Daher müssen gesundheitlich weniger bedenkliche Produkte für diesen Einsatzzweck gesucht werden. So werden auch in anderen Bereichen zunehmend physiologisch und epidemiologisch gesundheitlich bedenkliche oder aber für die Umwelt schädliche Substanzen, wie beispielsweise Antibiotika, formaldehydabspaltende Stoffe, halogenierte Substanzen, u.v.a.m. beispielsweise in Lebensmitteln, Futtermitteln, Haustierfutter, Silagen, Trester, oder anderen Abfällen aus der Lebensmittelindustrie, durch weniger bedenkliche Stoffe ersetzt. Der Zweck dieser Stoffe ist zum einen auf die Werterhaltung des eigentlichen Produktes gerichtet. Zum anderen aber soll auch deren hygienischer Zustand verbessert werden, bzw. eine verlängerte Haltbarkeit erzielt werden.

Es ist bekannt, dass Sorbinsäure zur Konservierung von Futtermitteln eingesetzt werden kann. Sorbinsäure (trans,trans-2,4-Hexadiensäure) ist eine farblose, feste Verbindung, die sich nur wenig in kaltem Wasser löst und weltweit als Konservierungsstoff verwendet wird. Das Wirkprinzip wird durch die Sorbinsäure in undissoziierter Form bestimmt. Die beste Wirkung entfaltet Sorbinsäure daher im sauren pH-Bereich. Sorbinsäure und seine Salze besitzen eine sehr gute mikrobiostatische, antimykotische Wirkung. Gleichzeitig ist Sorbinsäure als ungesättigte Fettsäure praktisch ungiftig, was sehr umfangreiche Daten belegen, und durch die jahrzehntelange Anwendung dieser Säure im Lebensmittelbereich, in Tierfuttern u.a. belegt ist.

Neben Sorbinsäure werden auch andere organische Säuren seit Jahren zur Konservierung von Futtermitteln und zur Verbesserung der Futterhygiene eingesetzt. Gerade im Futter für Jungtiere sind an die hygienische Qualität besondere Anforderungen zu stellen. Daher sind einige organischen Säuren als Futterzusatzstoffe, basierend auf den nationalen futtermittelrechtlichen Bestimmungen ohne Höchstmengenbegrenzung zugelassen. Diese Säuren wirken aber korrosiv und verursachen auf Grund ihrer Flüchtigkeit z. T. Geruchsbelästigungen und erfordern besondere Vorsicht im Umgang, wenn das Risiko einer aus Sicht des Gesundheitsschutzes unerwünschten inhalatorischen Aufnahme vermieden werden soll.

Probiotika werden in der Nutztierernährung eingesetzt um die Mikroorganismenzusammensetzung im gesamten Verdauungstraktes positiv zu beeinflussen (s. z.B. Asian-Aus. J. Anim. Sci. 2000, Heft 13, Nr. 1: 86-95). Die Leistungsförderung durch Probiotika erreicht jedoch nicht das Niveau der Fütterungsantibiotika. Im Gegensatz zu den Antibiotica handelt es sich dabei nicht um bestimmte Stoffwechselprodukte von Bakterien oder Pilzen, die hemmend oder abtötend auf die Mikroorganismen wirken, sondern es sind die zugeführten, in der Regel noch lebensfähigen Mikroorganismen selbst, die über eine veränderte Zusammensetzung der Mikroflora dem Nutztier zu höherer Leistung verhelfen können.

Die durch solche Zusätze erzielbaren Verbesserungen sind nicht so hoch wie bei antibiotisch wirksamen Stoffen. Größenordnungen von 3 bis 6 % Wachstumssteigerung (Zunahmen) und Verbesserung der Futterverwertung erscheinen realistisch (H. Jeroch et al., Ernährung landwirtschaftlicher Nutztiere (1999), S. 390). Zu den Faktoren, die sich nachteilig auf Gewichtszunahme und Futterverwertung auswirken können, gehört auch das Auftreten von Durchfällen. In diesem Zusammenhang stellten Kirchgessner et al. (Arch. Anim. Nutr., 1993, Vol. 44, S. 111-121) bei verschiedenen Dosierungen eines Bacillus cereus Präparates keinen signifikanten Einfluss auf die Durchfallhäufigkeit fest. Von andere Autoren hingegen wurde eine statistisch gesicherte Senkung der Durchfallhäufigkeit durch Zusätze von B. cereus toyoi (Iben und Leibetseder, Tierärztl. Mschr. 76 (1989), 363-366, Verlag Ostag Wien), B. licheniformis bzw. B. cereus (Kyriakis et al., Research in Veterinary Science 1999, 67, 223-228) und Enterococcus faecium (Männer und Spieler, Microecology and Therapy, Vol. 26, 243-256,1997) beschrieben. In der jüngsten Literatur (O.Simon, G Breves, Tagungsband 6. Tagung Schweine- und Geflügelernährung, 2000, S. 45-50) ist der Effekt nicht eindeutig und eine statistische Sicherung schwierig. So war nur in 14 von insgesamt 23 Versuchen eine Verbesserung um > 1 % Lebendmassenzunahme an Aufzuchtferkeln festzustellen.

Enzyme werden zu verschieden Zwecken in Tierfuttermitteln eingesetzt. Zu nennen sind insbesondere Enzyme welche andere antinutritive Futterinhaltsstoffe so stark abbauen, dass eine erhöhte Verfügbarkeit anderer Nährstoffe erreicht wird (z.B.: Pentosanasen, β-Glucanasen). Außerdem soll eine Lockerung von Zellwandstrukturen mit dem Ziel einer Verdaulichkeitserhöhung von Zellwandbestandteilen erreicht werden (z.B.: Cellulasen, β-Glucosidasen, Phytase). Weiterhin kann durch den Zusatz von Enzymen zum Tierfutter eine quantitative Unterstützung von körpereigenen Enzymen und damit eine verbesserte Verdauung erreicht werden (z.B.: Lipasen, Amylasen und Glucoamylasen, Carboxypeptidasen, Trypsin, Chymotrypsin, Elastase, Proteasen, Peptidasen).

So zeigte ein Zusatz von Xylanase (Gdala, J. et al., Anim. Feed Sci. Technol. 65 (1997) 15-33) eine wesentlich verbesserten Verdaulichkeit von Xylose, Arabinose und Mannose in der Ferkelernährung. Igbasan, F.A. et al. (6. Tagung Schweine- und Geflügelernährung, 2000, Tagungsband, S. 71-74) beschreiben in Ihren Untersuchungen Phytasen aus verschieden Bakterien wie Bacillus subtilis, Escherichia coli, welche eine bessere Aktivität als Pilzphytasen aufweisen.

Sorbinsäure als Futtermittelzusatz steigert zwar die Leistung in der Tierzucht hinsichtlich Zuwachsrate und Futterverwertung schon erheblich, die Verwertung der Futtermittel ist aber aufgrund des nach wie vor hohen Gehalts an nichtverdaulichen Bestandteilen noch nicht optimal. Es bestand das Bedürfnis nach einem Futtermittel mit leistungsfördernden Zusätzen ohne die Nachteile der heute üblicherweise verwendeten Stoffe fort.

Es bestand demnach die Aufgabe, einen einfach zu handhabenden leistungsfördernden Zusatz zur Verfügung zu stellen, der diese Nachteile nicht aufweist.

Gelöst wird diese Aufgabe durch ein Präparat (Zusammensetzung), welches Sorbinsäure sowie mindestens eine Kultur eines probiotisch wirkenden Mikroorganismus enthält. Bevorzugt ist ein Präparat, welches neben den genannten Inhaltsstoffen einen Träger enthält. Gleichermaßen wird die Aufgabe gelöst durch ein Futtermittelzusatzkit, welches getrennt aufeinander abgestimmte Mengen von Mikroorganismenkultur(en) und Sorbinsäure enthält.

Überraschenderweise zeigen die erfindungsgemäßen Präparate die oben beschriebenen Nachteile nicht. Die Präparate zeigen vielmehr gute Eigenschaften in der Handhabung. Zudem kann überraschenderweise auch ein positiver Einfluss auf die Wachstumsleistung von jungen Tieren schon in relativ geringen Sorbinsäuremengen festgestellt werden.

Unter Probiotika sollen lebensfähige Formen von Mikroorganismen oder Sporen verstanden werden, die dem Tier kontinuierlich mit dem Futter zugeführt werden können. Es handelt sich dabei um ausgewählte Hefestämme oder Milchsäurebakterien (morphologisch uneinheitliche grampositive, unbewegliche und katalasenegative Bakterien, wie Streptococcacaeae einschließlich der Bakterien der Gattung Enterococcus, Lactobacillaceae, Bacillaceae oder Actinomycetaceae). Es handelt sich dabei um Stämme die besonders säureresistent sind. Bei Sporenbildnern werden die Sporen als Futterzusatzstoff verwendet.

Bevorzugt werden folgende Mikroorganismen oder Kombinationen:
*Bacillus cereus*
(besonders *Bacillus cereus var. toyoi)*
*Bacillus clausii.*
*Bacillus licheniformis*
*Bacillus subtilis*
*Bifidobacterium bifidum*
*Bifidobacterium breve*
*Bifidobacterium infantis*
*Bifidobacterium lactis*
*Bifidobacterium longum*
*Bifidobacterium adolescentis*
*Enterococcus faecium*
*Enterococcus mundtii*
*Lactobacillus acidophilus*
*Lactobacillus amylovorus*
*Lactobacillus bulgaricus*
(besonders *Lactobacillus delbrueckii subsp. bulgaricus)*
*Lactobacillus casei*
*Lactobacillus crispatus*
*Lactobacillus farciminis*
*Lactobacillus gallinarum*
*Lactobacillus gasseri*
*Lactobacillus johnsonii*
*Lactobacillus paracasei*
*Lactobacillus plantarum*
*Lactobacillus reuteri*
*Lactobacillus rhamnosus*
*Lactobacillus salivarius*
*Pediococcus acidilactici*
*Saccharomyces cerevisiae*
*Streptococcus infantarius*
*Streptococcus thermophilus*
(besonders *Streptococcus salivarius subsp. thermophilus).*

Erfindungsgemäß enthält das Futtermittel >0 bis 20 g/kg Futtermittel Sorbinsäure, bevorzugt 5,0 bis 15,0 g/kg Futtermittel, besonders bevorzugt 7,5 bis 12,5 g/kg Futtermittel. Die Sorbinsäure ist in den erfindungsgemäßen Präparaten in Mengen von 90,0 bis 99,9 Gew.-%, bevorzugt 95,0 bis 99,9 Gew.-% enthalten. Die Konzentration der erfindungsgemäßen Präparate im Futtermittel liegen bei > 0,0 bis 2,0 Gew.-%, bevorzugt bei 0,5 bis 1,5 Gew.-%.

Die Mikroorganismen (Probiotika) oder Kombinationen derselben werden in den erfindungsgemäßen Präparaten in Mengen eingesetzt, die 10⁷ bis 10¹⁰, vorzugsweise 0,1 - 50 x 10⁹, lebensfähigen Mikroorganismen je kg Futtermittel entsprechen.

Als Träger sowohl für die Sorbinsäure als auch für das Probiotikum können organische oder anorganische Materialien verwendet werden, insbesondere solche, die wasserunlöslich und gegenüber den eingesetzten Mikroorganismen inert sind oder ihre Lebensfähigkeit nicht beeinträchtigen. Dazu zählen beispielsweise Stärke und andere Polysaccharide wie Cellulose.

Ein erfindungsgemäßes Präparat wird hergestellt, indem man eine Sporen enthaltende Mikroorganismenkultur, gegebenenfalls immobilisiert auf einem Träger oder verkapselt und die Sorbinsäure mechanisch gleichmäßig durchmischt. Im Falle lebender Kulturen insbesondere bei der Verwendung von Milchsäurebakterien ist es zweckmäßig diese gegen mechanische und thermische Einflüsse bei Transport und Lagerung zu schützen. Dazu werden die Mikroorganismen mit Mikrokapseln /Mikrospheren versehen und widerstehen damit unerwünschten Einflüssen durch Verdauungssäfte. Hierbei ist es möglich, die Sorbinsäure getrennt von den Milchsäurebakterien in die Mikrosphere oder aber in eine der äußeren Hüllschichten einer Mikrokapsel so unterzubringen, dass Sorbinsäure früher abgelöst wird und beispielsweise im Magen bereits zu einer merklichen pH-Absenkung führt, die Mikroorganismen aber erst später im Magen-Darm -Trakt freigesetzt werden . Auch eine Mischung von verkapselten Mikroorganismen und Sorbinsäure ist möglich. Für die Verkapselung eignet sich beispielsweise Gelatine, Lecithine, Stearate, Alginate, Traganth, Xanthan, Carrageen, Cassia-Gum, Gummi arabicum, Maltodextrine, modifizierte Stärken, Cellulosen, Mono- und Diglyceride von Speisefettsäuren verestert mit organischen Säuren oder nicht verestert, Palmitin oder Mischungen daraus. Weiterhin können die Mikroorganismen gegebenenfalls auf einem Träger immobilisiert werden und die Sorbinsäure getrennt zur Verfügung gestellt werden. Dazu müssen beide nacheinander gleichmäßig in das Futtermittel eingemischt werden. Die Immobilisierung der Mikroorganismen kann beispielsweise durch Aufsprühen von Kulturlösungen auf entsprechende Träger erfolgen.

Die natürliche Versporung der Bacillus-Probiotika bietet einen guten Schutz gegenüber äußeren Einflüssen. Dadurch bleibt die Wirksamkeit dieser Mikroorganismen gewährleistet. Eine Kombination mit Sorbinsäure erfordert, dass diese Versporung bei der Herstellung besonders hohe Produktqualität aufweist.

Durch den Sorbinsäurezusatz wird die Stabilität fester Futtermittel bei der Lagerung und beim Pelletieren verbessert. Optimiert werden die Mischungen durch ein Aufsprühen aufgebracht.

Als Tierfuttermittel geeignet sind z. B. Grünfutter, Silagen, Trockengrünfutter, Wurzeln, Knollen, fleischige Früchte, Körner und Samen, Biertreber, Trester, Bierhefe, Destillationsschlempe, Müllereinebenerzeugnisse, Nebenerzeugnisse der Zucker-, Stärkeherstellung und Ölgewinnung und verschiedene Lebensmittelabfälle. Solchen Futtermitteln können bestimmte Futtermittelzusatzstoffe (z.B. Antioxidantien) oder Mischungen aus verschiedenen Substanzen (z.B. Mineralstoffmischungen, Vitaminmischungen) zur Verbesserung beigegeben werden. Spezielle Futtermittel werden auch für bestimmte Tierarten und deren Entwicklungsstadium angepasst. Dies ist beispielsweise in der Ferkelaufzucht der Fall. Hier werden Saugferkelfutter und Ferkelaufzuchtfutter verwendet. Das erfindungsgemäße Präparat kann direkt dem Tierfuttermittel bzw. auch in Abmischung mit anderen Futtermittelzusatzstoffe oder aber über Vormischungen dem eigentlichen Futtermittel zugegeben werden. Das Präparat kann trocken dem Futter zugemischt werden, vor einer weiteren Verarbeitung (z.B. Extrusion) zugegeben werden bzw. im Gemisch dispergiert zudosiert werden. Darüber hinaus können auch die Einzelbestandteile des Präparats getrennt einzelnen Bestandteilen des Futtermittels zugegeben werden, wenn nicht höhere Temperaturen auftreten, bei denen die Lebenstätigkeit der Mikroorganismen beeinträchtigt werden kann.

Das Präparat kann als alleiniger Zusatzstoff zu Tierfuttermitteln beispielsweise für die Kälber- oder Lämmeraufzucht, besonders bevorzugt zu Saugferkelfutter (Prestarter) und Ferkelaufzuchtfutter (Starterfutter) zugegeben werden oder in Abmischung mit anderen Futtermittelzusatzstoffen für diese Tiere angewendet werden.

Das erfindungsgemäße Präparat ist in der Lage, den hygienischen Status zu verbessern, dadurch dass erwünschte Mikroorganismen von vornherein günstige Entwicklungsbedingungen vorfinden, während unerwünschte Organismen und Verderbserreger unterdrückt werden, die anderenfalls vorhandene Nährstoffe verbrauchen können.

Teile der Mikroorganismenpopulationen des Futtermittels erreichen trotz des sauren Milieus im Magen, der Gallensalze und proteolytischen Enzyme im Dünndarm den Dickdarm. Aus dem Einsatz von Probiotika ergibt sich somit primär eine Prophylaxe gegen infektiöse Magen-Darm-Erkrankungen. Das Unterdrücken unerwünschter Mikroorganismen im Futter wie im Magen-Darmtrakt der Tiere unterstützt diesen Effekt. Hinzu kommt die Bildung von Milchsäure und niederen Fettsäuren, die sich gleichfalls hemmend auf die Entwicklung pathogener Keime auswirken.

Die komplexen Nährstoffbedürfnisse vieler probiotischer Mikroorganismen können durch den Einsatz von Enzymen, die höher molekulare Inhaltsstoffe der Futtermittel aufschließen und solche Stoffe freisetzen, zusätzlich erfüllt werden, wodurch diese Organismen gegenüber den unerwünschten Mikroorganismen einen Vorteil erlangen.

Erfindungsgemäß werden unter dem Begriff Enzyme biologische Katalysatoren mit proteinogener Struktur verstanden, welche mit Hilfe von Mikroorganismen durch Fermentation oder aus Pflanzenteilen durch Extraktion oder Anreicherung gewonnen werden. Oft werden keine reinen Enzyme, sondern angereicherte Enzympräparate in Form von Mischungen in verschiedener Zusammensetzung und Aktivität gewonnen. Enzyme reagieren substratspezifisch, d.h. dass ein Enzym auch nur eine Substanz(klasse) angreifen kann. Beispielsweise kann das Enzym Phytase, Phytinsäure (durch die Abspaltung von Phosphatresten) angreifen; dadurch wird verwertbarer Phosphor freigesetzt und die chelatisierende Wirkung von Phytinsäure auf Ca-, Mg-, Fe- und Zn-lonen, die als Spurenelemente im Futter wichtig sind, unterbunden.

Bevorzugt werden die Enzyme aus den jeweiligen Klassen, welche eine hohe Lagerstabilität, breites pH- und Temperaturoptimum, Pelletierungsmöglichkeiten mit Tierfuttern und einer möglichen Passage bis zum Darmtrakt der Tiere verfügen.

Beispiele für erfindungsgemäß einsetzbare Enzyme/Enzympräparate sind (mit bevorzugter mind. Enzymaktivitäten/kg Futter):
- Phytase (Schweinen/Ferkeln zweckmäßigerweise mind. 500 FTU, bei Geflügel wie Legehennen, Puten zweckmäßigerweise mind. 300 FTU und anderen Geflügelarten zweckmäßigerweise mind. 500 FTU)
- Beta-Glucanasen (z.B. Endo-1,4-Beta-Glucanase, Endo-1,3(4)-Beta-Glucanase zweckmäßigerweise mit 400 bis 600 BGU )
- Endo-1,4-Beta-Xylanase (zweckmäßigerweise mit 500 bis 850 EXU )
- Cellulasen (zweckmäßigerweise 900 bis 2000 Hemicellulaseaktivität ****)
- Alpha-Amylase (zweckmäßigerweise mind. 250 Amylaseaktivität)
- Alpha-Galactosidase (zweckmäßigerweise mind. 200 Galactosidaseaktivität****)
- Pentosanasen (zweckmäßigerweise mind. 200 Pentosanaseaktivität ****)
- Beta- Glucosidasen (zweckmäßigerweise mind. 250 Glucosidaseaktivität ****)

*1 FTU setzt 1 mmol anorganischen Phosphor/min aus 0,0051 mol/l Na-phytat, bei pH 5,5 und 37° C frei.
**1 BGU setzt 0,278 µmol reduzierende Zucker (als Glucose-äquivalente)/min aus einer 0,5 %-igen β-Glucan Lösung bei pH 3,5 bei 40 ° C frei
***1 EXU setzt 1,0 µmol reduzierende Zucker (als Xylose-äquivalente)/min aus einer 1,0 %-igen Xylan-Lösung bei pH 3,5 bei 40 ° C frei
****Standard FCC oder AATCC-Methoden.

Weiterhin finden Anwendung Enzyme wie Glucoamylasen, Glucoseoxidasen, verschiedene Lipasen, Mannase (endo-1,4-β), Polygalacturonasen, Transglutaminasen und Xylanasen, verschiedener Aktivitäten und Einsatzkonzentrationen, in Abhängigkeit von den Aktivitäten der Tiere.

Die Enzyme können auch an Trägern in gebundener Form oder als Mischung aus verschiedenen Herstellungsprozessen vorliegen.

Die Dosierungen der Enzyme oder Enzympräparate werden nach den vorliegenden Enzymaktivitäten ausgerichtet und dabei so gewählt, dass der erforderliche Aufschluss der Inhaltsstoffe oder die Inaktivierung unerwünschter Substanzen zuverlässig vor der Verfütterung oder einer evtl. Weiterverarbeitung erreicht sind.

Überraschenderweise wurde gefunden, dass schon durch Zusatz geringer Mengen erfindungsgemäßer Präparate in der Ferkelaufzucht eine deutliche Leistungsverbesserungen hinsichtlich Zuwachsrate und Futterverwertung erreicht werden kann. Weiterhin eignen sich Futtermittel mit dem erfindungsgemäßen Präparat als Milchaustauscher bei der Frühentwöhnung von Sauglämmern oder Kälbern.

Die Erfindung wird nachfolgend anhand von Beispielen erläutert.

### Beispiele

### Beispiel 1

0,01 kg Sporen von *Bacillus cereus* werden mit 0,99 kg Sorbinsäure in einem Tellermischer trocken vermischt, so dass keine mechanischen Schäden an der Sporenoberfläche auftreten, aber eine gleichmäßige Vermischung erreicht wird. Diese Mischung wird mit 100 kg Ferkelfutter folgender Zusammensetzung vermischt (Angaben in Gew.-%).

| | |
|---|---|
| Sojaextraktionsschrot | 22,00 |
| Gerste | 40,00 |
| Weizen | 31,00 |
| Planzenöl | 2,90 |
| L-Lysin - HCI | 0,40 |
| DL-Methionin | 0,10 |
| L-Threonin | 0,10 |
| Mineralfutter | 3,50 |

Mit diesem Futter wurde eine deutliche Leistungssteigerung bei der Ferkelaufzucht erzielt.

### Beispiel 2

Etwa 0,01 - 0,05 kg (entsprechend einer Konzentration von mindestens 5 x 10⁹ lebenden Mikroorganismen) eines handelsüblichen verkapselten Präparates, bestehend aus *Lactobacillus rhamnosus* und *Enterococcus faecium* werden mit 0,75 kg Sorbinsäure in einem Doppelkonusmischer mit Taumelbewegungen für etwa 15 min gemischt. Die homogene Mischung wird mit 100 kg Ferkelfutter folgender Zusammensetzung vermischt (Angaben in %).

| | |
|---|---|
| Fischmehl | 4,00 |
| Sojaextraktionsschrot | 18,50 |
| Gerste | 40,00 |
| Weizen | 33,00 |
| Planzenöl | 1,90 |
| L-Lysin - HCI | 0,2 |
| DL-Methionin | 0,1 |
| L-Threonin | 0,1 |
| Mineralfutter | 2,2 |

Mit diesem Futter wurde eine deutliche Leistungssteigerung bei der Ferkelaufzucht erzielt.

## Patentansprüche

1. Präparat, welches Sorbinsäure und mindestens eine Kultur eines probiotisch wirkenden Mikroorganismus (= Probiotikum) enthält.

2. Präparat nach Anspruch 1, **dadurch gekennzeichnet, dass** das Präparat mindestens 90 Gew.-% Sorbinsäure enthält.

3. Präparat nach einem der Ansprüche 1 oder 2, **dadurch gekennzeichnet, dass** es ein Probiotikum oder Probiotika in einer Menge enthält, die 10⁷ bis 10¹⁰ lebensfähigen Mikroorganismen je g des aus dem Präparat hergestellten Futtermittels entspricht.

4. Präparat nach einem der Ansprüche 1 bis 3, **dadurch gekennzeichnet, dass** das Probiotikum ausgewählt wird aus:
*Bacillus cereus*, *Bacillus clausii, Bacillus licheniformis, Bacillus subtilis, Bifidobacterium bifidum, Bifidobacterium breve, Bifidobacterium infantis, Bifidobacterium lactis, Bifidobacterium longum, Bifidobacterium adolescentis, Enterococcus faecium, Enterococcus mundtii, Lactobacillus acidophilus, Lactobacillus amylovorus, Lactobacillus bulgaricus, Lactobacillus casei, Lactobacillus crispatus, Lactobacillus farciminis, Lactobacillus gallinarum, Lactobacillus gasseri, Lactobacillus johnsonii, Lactobacillus paracasei, Lactobacillus plantarum, Lactobacillus reuteri, Lactobacillus rhamnosus, Lactobacillus salivarius, Pediococcus acidilactici, Saccharomyces cerevisiae, Streptococcus infantarius, Streptococcus thermophilus* und Kombinationen derselben.

5. Präparat nach einem der Ansprüche 1 bis 4, **dadurch gekennzeichnet, dass** es zusätzlich mindestens ein Enzym und/oder Enzympräparat enthält.

6. Präparat nach Anspruch 5, **dadurch gekennzeichnet, dass** das Enzym ausgewählt wird aus:
Phytase, Cellulase, Glucanase, Hemicellulase (Xylanase), Amylase, Galaktosidase, Pentosanase, Glucosidase, verschiedene Lipasen, Mannase (endo-1,4-β), Polygalacturonase, Transglutaminase und Mischungen derselben.

7. Futtermittelzusatzkit, welches getrennt voneinander
- eine oder mehrere Mikroorganismenkulturen (= Probiotikum) und
- Sorbinsäure enthält.

8. Futtermittel, **dadurch gekennzeichnet, dass** es ein Präparat nach Anspruch 1 enthält.

9. Futtermittelzusatz, **dadurch gekennzeichnet, dass** es ein Präparat nach Anspruch 1 enthält.

10. Futtermittel nach Anspruch 8, **dadurch gekennzeichnet, dass** es >0,0 bis 2,0 Gew.-% (bezogen auf das Futtermittel) des Präparates enthält.

11. Verwendung des Präparates nach Anspruch 1 als Tierfutter- oder Futtermittelzusatz.

12. Verwendung nach Anspruch 11 in der Schweineaufzucht.

13. Verwendung nach Anspruch 11 in der Rinderaufzucht.

14. Verwendung nach Anspruch 11 in der Lämmeraufzucht.

15. Verwendung nach Anspruch 11 in der Geflügelaufzucht.
